# EUROPEAN PATENT APPLICATION

(11) **EP 2 505 575 A1**
(43) Date of publication of application: **03.10.2012**
(21) Application number: 10833158.8
(22) Date of filing: 19.11.2010
(51) Int. Cl.: C07C 43/225, C08G 65/323, C10M 107/38, C10N 20/04, C10N 30/00, C10N 30/06, C10N 40/18

(54) **ETHER COMPOUND, LUBRICANT CONTAINING SAME, AND COMPOSITION FOR LUBRICANT CONTAINING SAME**

(30) Priority: 26.11.2009 JP 2009268683
(71) Applicant: Asahi Glass Company, Limited, Tokyo 100-8405 (JP)
(72) Inventor: SHIRAKAWA, Daisuke, Tokyo 100-8405 (JP); GOZU, Yoshiyuki, Tokyo 100-8405 (JP)
(74) Representative: Müller-Boré & Partner Patentanwälte
(86) International application number: PCT/JP2010/070748
(87) International publication number: WO 2011/065312

(57) **Abstract**

To provide an ether compound which is capable of maintaining high adhesion to a base material even in the presence of moisture, and a lubricant and a composition for a lubricant, containing the ether compound.

An ether compound represented by (X-)ₛ(W-)ₜ(Z-)ᵤY is used. X = a group having the following formula (X0) at the terminal thereof, W = a group having HO-CH₂CF₂O- at the terminal thereof, Z = a group having CF₃(CF₂)ₐ₃O- at the terminal thereof, Y = a (s+t+u) valent perfluorinated hydrocarbon group or the like, x = 1 to 7, w = 0 to 4, z = 0 to 2, (s+t+u) = 2 to 7, a3 = 0 to 19, and R¹ to R⁵= a hydrogen atom, a fluorine atom, etc.

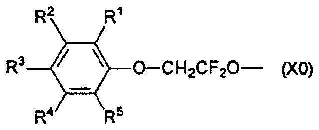

## Description

### TECHNICAL FIELD

The present invention relates to a fluoroether compound, a lubricant containing the same, and a composition for a lubricant containing the same.

### BACKGROUND ART

As an external storage device, it is common to employ an external storage device having a system (CSS system) in which a hard disk having a recording medium layer is rotated at a high speed to operate a recording or reading element (head). The hard disk may, for example, be a fixed type magnetic disk, an optical disk or a magnetooptical disk, and the fixed type magnetic disk is most widely prevalent as a hard disk.

In order to increase the capacity of an external storage device, it is necessary to increase the surface recording density of a recording medium layer. For such a purpose, it is necessary to make an interval between a head and a hard disk narrower so as to reduce a bit size. Along with this, it is necessary to increase the smoothness of the surface on a hard disk. However, if the smoothness of the surface on a hard disk is increased, a head will be readily adsorbed on the surface of a hard disk.

Further, in order to achieve the high responsibility, it is also necessary to increase the rotational speed of a hard disk. However, if the rotational speed is increased, e.g. the contact probability or the abrasion between a hard disk and a head increases.

In order to solve the problems, a lubricant as one of surface-treating agents is applied on the surface of a hard disk. As the lubricant, the following has been known:
(1) A polyfluorinated polyether compound (hereinafter, referred to as "PFPE") having four hydroxy groups at the terminals thereof (Patent Document 1)

However, the PFPE of (1) has the following problems resulting from the hydroxy groups.

Since the proportion of the hydroxy groups in all terminal groups is high, the PFPE easily adsorbs the moisture, and by the adsorption of the moisture, the adhesion to the surface of a hard disk deteriorates.

An organic substance having affinity with the hydroxy groups is easily included.

If a compound having a polar group is present, the polar group and the hydroxy group are reacted to deteriorate the adhesion to the surface of a hard disk.

On the other hand, as a PFPE having no hydroxy groups at the terminals thereof, the following has been proposed (Patent Document 2).
(2) PFPE having three vinyl groups at the terminals thereof, which are aligned by means of ultraviolet irradiation so as to achieve adhesion to the surface of a hard disk (Patent Document 2)

### PRIOR ART DOCUMENTS

### PATENT DOCUMENTS

- Patent Document 1:: WO 2005/068534
- Patent Document 2:: JP-A-2009-149835

### DISCLOSURE OF INVENTION

### TECHNICAL PROBLEM

However, according to studies by the present inventors, it has been confirmed that even in the case of the PFPE of (2), the adhesion to the surface of a hard disk tends to easily deteriorate in the presence of moisture.

It is an object of the present invention to provide an ether compound which is capable of maintaining high adhesion to a base material even in the presence of moisture, and a lubricant and a composition for a lubricant, containing the ether compound.

### SOLUTION TO PROBLEM

The present invention provides the following [1] to [11]:
[1] An ether compound represented by the following formula (A):

   (X-)ₛ (W-)ₜ (Z-)ᵤ Y (A)

   wherein X is a group (X) having a group represented by the following formula (X0) at the terminal thereof,
   W is a group (W) having a group represented by the following formula (W0) at the terminal thereof,
   Z is a group (Z) having a group represented by the following formula (Z0) at the terminal thereof,
   Y is a group (Y) which is a (s+t+u) valent perfluorinated hydrocarbon group or a group having an etheric oxygen atom inserted between carbon-carbon atoms of the hydrocarbon group,
   s is an integer of from 1 to 7,
   t is an integer of from 0 to 4,
   u is an integer of from 0 to 2, and
   (s+t+u) is an integer of from 2 to 7:

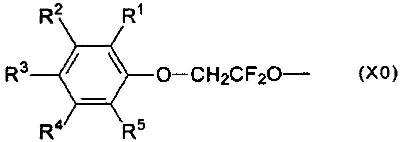

   HO-CH₂CF₂O- (W0)

   CF₃(CF₂)ₐ₃O- (Z0)

   wherein among R¹ to R⁵, two of them are each independently an atom or group selected from a hydrogen atom, a fluorine atom, a methyl group, a trifluoromethyl group, a phenyl group, a perfluorophenyl group and a hydroxy group, and other three are each independently a hydrogen atom or a fluorine atom, and
   a3 is an integer of from 0 to 19.
[2] The ether compound according to [1], wherein the group (X) is a group represented by the following formula (X1), the group (W) is a group represented by the following formula (W1), and the group (Z) is a group represented by the following formula (Z1):

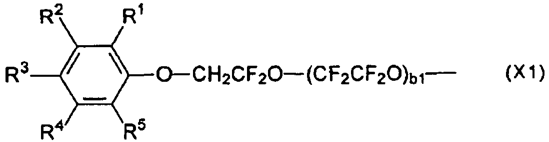

   HO-CH₂CF₂O-(CF₂CF₂O)_{b2} - (W1)

   CF₃(CF₂)ₐ₃O-(CF₂CF₂O)_{b3} - (Z1)

   wherein b1 to b3 are each independently an integer of from 3 to 200.
[3] The ether compound according to [2], wherein the group (X1) is a group selected from the group consisting of groups represented by the following formulae (X1-1) to (X1-5): 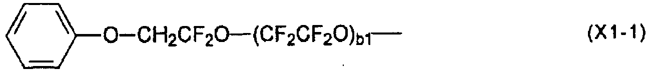 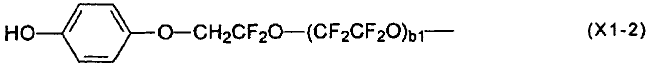 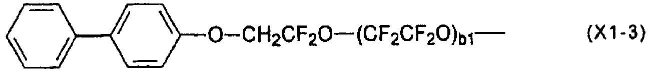 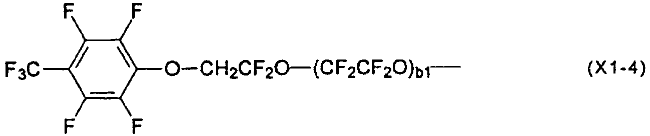 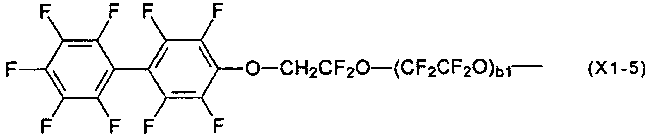
[4] The ether compound according to [1] to [3], wherein the group (Y) is a group selected from the group consisting of groups represented by the following formulae (Y-1) to (Y-8): 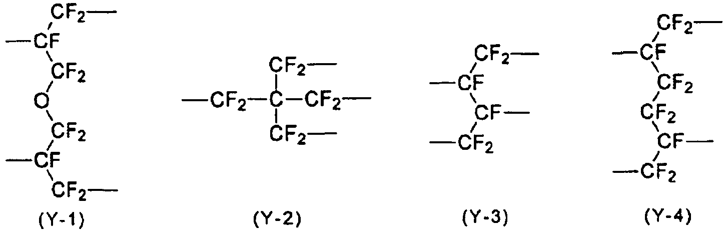 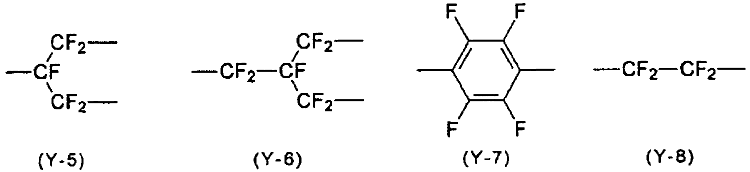
[5] The ether compound according to [1] to [4], which has no -OCF₂O- structure.
[6] The ether compound according to [1] to [5], wherein the average value of s/(s+t+u) is from 0.25 to 1.0.
[7] The ether compound according to [1] to [6], wherein the number average molecular weight (a value calculated as a perfluoropolyether) is from 500 to 1,000,000, and further the molecular weight distribution (a value calculated as a perfluoropolyether) is from 1.01 to 1.50.
[8] A lubricant containing the ether compound as defined in [1] to [7], wherein the proportion of the ether compound is at least 10 mass%, per 100 mass% of the lubricant (excluding a liquid medium).
[9] The lubricant according to [8], which is used for magnetic recording media.
[10] A composition for a lubricant, comprising the ether compound as defined in any one of [1] to [7] and a liquid medium.
[11] The composition for a lubricant according to [10], wherein the proportion of the ether compound is from 0.0001 to 10 mass%, per 100 mass% of the composition for a lubricant.

### ADVANTAGEOUS EFFECTS OF INVENTION

According to the ether compound of the present invention, it is possible to maintain high adhesion to a base material even in the presence of moisture.

According to the lubricant of the present invention, it is possible to maintain high adhesion to a base material even in the presence of moisture.

According to the composition for a lubricant of the present invention, it is possible to form a lubricant coating which can maintain high adhesion to a base material even in the presence of moisture.

### DESCRIPTION OF EMBODIMENTS

In this specification, a compound represented by the formula (A-1) will be referred to as a compound (A-1). The same also applies to compounds represented by other formulae.

Further, a group represented by the formula (X0) will be referred to as a group (X0). The same also applies to groups represented by other formulae.

### <Ether compound>

In the present invention, an ether compound represented by the following formula (A) will be referred to as an ether compound (A) or a compound (A).

(X-)ₛ(W-)ₜ(Z-)ᵤ Y (A)

The compound (A) is a compound in which s number of groups (X), t number of groups (W) and u number of groups (Z) are bonded to the group (Y). s is an integer of from 1 to 7, t is an integer of from 0 to 4, u is an integer of from 0 to 2, and (s+t+u) is an integer of from 2 to 7. That is, in the compound (A), at least one group (X) is bonded to the di- to heptavalent group (Y). The group (W) and the group (Z) are groups which are optionally bonded.

s is an integer of from 1 to 7, preferably an integer of from 1 to 4.

t is an integer of from 0 to 4, preferably 0 or 1.

u is an integer of from 0 to 2, preferably 0 or 1.

(s+t+u) is an integer of from 2 to 7, preferably from 2 to 4. That is, the group (Y) is preferably a di- to tetravalent group.

### (Group (X))

The group (X) is a group having the following group (X0) at the terminal thereof:

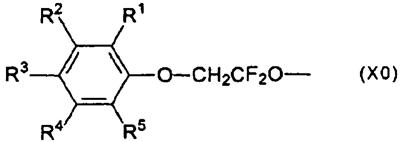

Among R¹ to R⁵, two of them are each independently a hydrogen atom, a fluorine atom, a methyl group, a trifluoromethyl group, a phenyl group, a perfluorophenyl group or a hydroxy group, and the other three are each independently a hydrogen atom or a fluorine atom

The group (X) is preferably a group (X1):

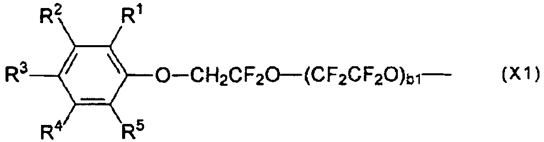

b1 is an integer of from 3 to 200, preferably an integer of from 3 to 30, more preferably an integer of from 5 to 20.

The group (X1) is preferably a group selected from the group consisting of the groups (X1-1) to (X1-5), more preferably the group (X1-1) or (X1-4), from the viewpoint of availability of the compound. In a case where a plurality of groups (X1) are present in one molecule, it is particularly preferred that such groups (X1) consist solely of the groups (X1-1) or the groups (X1-4).

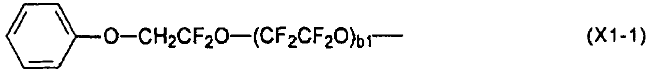

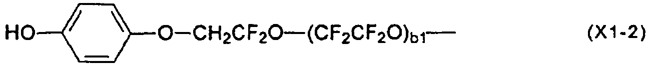

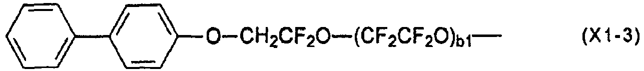

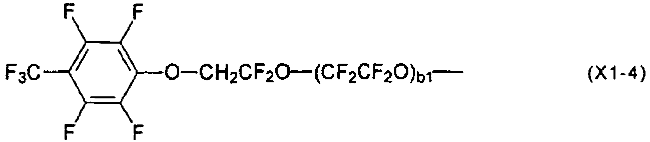

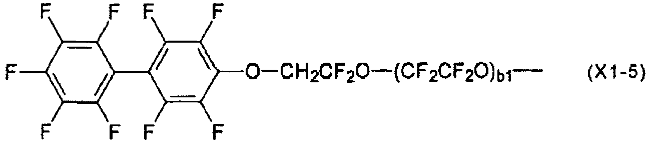

In a case where a plurality of the groups (X) are present in one molecule, they may be the same group or different groups. In a category where the groups (X) are the same group, groups differing in the number of structural units are also included. For example, the groups (X1) differing only in the number of b1 are considered to be the same group. In the case where a plurality of groups (X) are present in one molecule, the groups (X) are preferably the same group from the viewpoint of production efficiency and the effect of the present invention.

### (Group (W))

The group (W) is a group having the group (W0) at the terminal thereof:

HO-CH₂CF₂O- (W0)

The group (W) is preferably a group (W1):

HO-CH₂CF₂O-(CF₂CF₂O)_{b2} - (W1)

b2 is an integer of from 3 to 200, preferably an integer of from 3 to 30, more preferably an integer of from 5 to 20.

The group (W) has a hydroxy group, whereby it is a group capable of contributing to the improvement of the adhesion to a base material. For example, in the case of heterogeneous binding of the compound (A) with a base material by the group (X) by means of ultraviolet irradiation, it is possible to improve the adhesion to the base material by the hydroxy group of the compound (A) having the group (W).

In a case where a plurality of groups (W) are present in one molecule, they may be the same group or different groups. In a category where the groups (W) are the same group, groups differing in the number of structural units are also included. For example, the groups (W1) differing only in the number of b2 are considered to be the same group. In the case where a plurality of groups (W) are present in one molecule, the groups (W) are preferably the same group from the viewpoint of production efficiency and the effect of the present invention.

### (Group (Z))

The group (Z) is a group having the group (Z0) at the terminal thereof:

CF₃(CF₂)ₐ₃O- (Z0)

a3 is an integer of from 0 to 19, preferably an integer of from 3 to 30, more preferably an integer of from 5 to 20.

The group (Z) is a group having -CF₃ at the terminal thereof, which is capable of contributing to lowering of the friction coefficient. It is preferred that the group (Z) has a chain length to a certain extent, from the viewpoint of high degree of freedom of -CF₃ in the molecule.

The group (Z) is preferably a group (Z1):

CF₃(CF₂)ₐ₃O-(-CF₂CF₂O)_{b3}- (Z1)

b3 is an integer of from 3 to 200, preferably an integer of from 3 to 30, more preferably an integer of from 5 to 20.

In a case where a plurality of groups (Z) are present in one molecule, they may be the same group or different groups. In a category where the groups (Z) are the same group, groups differing in the number of structural units are also included. For example, the groups (Z1) differing only in the number of a3 or b3 are considered to be the same group. In the case where a plurality of groups (Z) are present in one molecule, the groups (Z) are preferably the same group from the viewpoint of production efficiency and the effect of the present invention.

### (Group (Y))

The group (Y) is a (s+t+u) valent perfluorinated hydrocarbon group or a group having an etheric oxygen atom inserted between carbon-carbon atoms of the hydrocarbon group. The group (Y) preferably has no -CF₃. In a case where the group (Y) has -CF₃, the -CF₃ is preferably bonded to a quaternary carbon atom. The quaternary carbon atom means a carbon atom bonded to other four carbon atoms.

In a case where the group (Y) is the group having an etheric oxygen atom inserted between carbon-carbon atoms, the number of an etheric oxygen atom is preferably from 1 to 3. Since the etheric oxygen atom is present between carbon-carbon atoms, an etheric oxygen atom is not present at the terminals of the group (Y) bonded to the group (X), the group (W) and the group (Z).

The group (Y) may have a symmetrical structure or an asymmetrical structure. The group (Y) preferably has an asymmetrical structure. When the structure is asymmetric, the crystallinity of the compound (A) becomes low, and in a case where the compound (A) is used as a composition for a lubricant as dissolved in a liquid medium for example, the compound (A) is readily dissolved in the liquid medium, and further the viscosity of the composition for a lubricant becomes low, whereby the operation efficiency becomes good at the time of applying it on a base material. Here, the symmetrical structure of the group (Y) means an axisymmetrical structure in a plane, centered around the group (Y). Since the main chain is linearly present, the crystallinity tends to be high.

The group (Y) is preferably at least one selected from the group consisting of the groups (Y-1) to (Y-8), and in view of the asymmetrical structure as mentioned above, the group (Y-1) and the groups (Y-3) to (Y-6) are more preferred, and the group (Y-1) is particularly preferred.

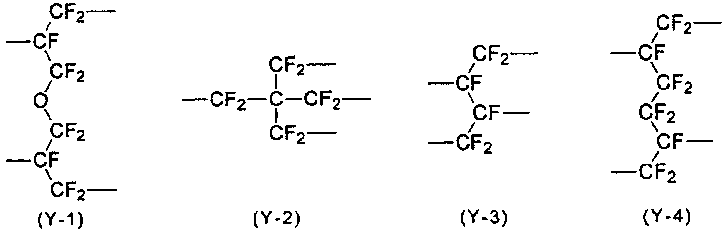

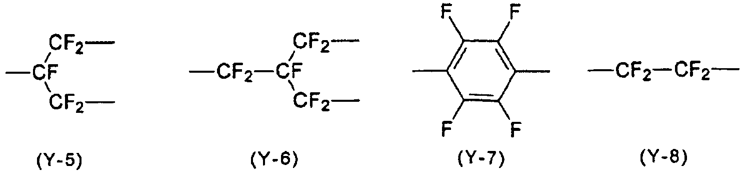

### (Compound (A))

The compound (A) preferably has no -OCF₂O- structure from the viewpoint of chemical stability. That is, it is preferred that the group (X), the group (Z), the group (W) and the group (Y) are a group having no -OCF₂O- structure, and it is also preferred that -OCF₂O- is not present in a structure of the compound (A). A fluorinated polyether having a -OCF₂O- structure has low chemical stability and is easily decomposable.

Such a compound having no -OCF₂O- structure means a compound in which the presence of such a structure cannot be detected by a conventional analysis (such as ¹⁹F-NMR). The presence of the -OCF₂O- structure can be confirmed by the presence of a peak of σ at from -50.0 to -56.0 ppm in ¹⁹F-NMR (solvent: CDCl₃) (however, other structures sometimes overlap with this peak). Further, the presence of the fluorinated polyether having a -OCF₂O- structure can also be confirmed as it is easily decomposed in the presence of a decomposition promoting catalyst.

The -OCF₂O- structure is a structure which is easily contained in a fluorinated ether compound having a poly(perfluorooxypropylene) chain, and this structure is not usually contained in the compound (A) having a (CF₂CF₂O)_{b1} chain. In the case of synthesizing the compound (A) by a direct fluorination reaction of a raw material compound having a (CH₂CH₂O)_{b1} chain, as a starting material, to form a (CF₂CF₂O)_{b1} chain, as an after-mentioned preferred production process, the resulting compound (A) has no -OCF₂O- structure so long as the raw material compound has no -OCH₂O-structure or -OCF₂O- structure. Further, in the process for producing the compound (A) employing such a direct fluorination reaction, a fluorinated ether compound having a -OCF₂O- structure will not be formed as a by-product.

In the production of the compound (A), a mixture of the compounds (A) differing in the values of s, t and u is usually produced. As the case requires, it is possible to selectively produce a mixture containing a specific compound (A) at a high proportion. Further, from the mixture of the compounds (A) differing in the values of s, t and u, it is also possible to separate one having a specific compound (A) at a high proportion.

A mixture of the compound (A) is preferably one having an average ratio of the group (X) in the mixture, i.e. an average value of s/(s+t+u), being from 0.25 to 1.0. The ratio of the group (X) being 1.0 means that the entire amount of the mixture substantially consists of the compound (A) having only the group (X). The average value of s/(s+t+u) being 0.25 to 1.0 means that the proportion of the group (X) is from 25 to 100 mol% per 100 mol% in total of the group (X), the group (W) and the group (Z) in the mixture of the compounds (A). It is possible to determine the proportions of the group (X), the group (W) and the group (Z) by means of a known method from the measurement results of ¹⁹F-NMR and ¹H-NMR.

When the above ratio of the group (X) is at least 0.25, the moisture is less likely to be adsorbed, and the adhesion to a base material increases. Further, the above ratio of the group (X) is more preferably from 0.5 to 1.0, further more preferably from 0.75 to 1.0, particularly preferably from 0.95 to 1.0.

The compound (A) is preferably the following compound (A-1) or (A-2).

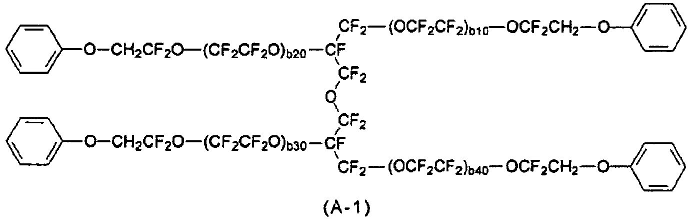

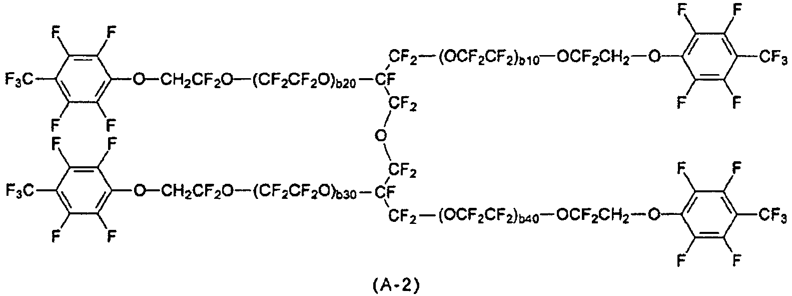

b10 to b40 correspond to the above-mentioned b1, and each of them is an integer of from 3 to 200, preferably an integer of from 3 to 30, more preferably an integer of from 5 to 20.

The number average molecular weight (hereinafter referred to as Mn, a value calculated as a perfluoropolyether) of the compound (A) is preferably from 500 to 1,000,000, more preferably from 1,000 to 10,000.

The molecular weight distribution (hereinafter referred to as Mw/Mn, a value calculated as a perfluoropolyether) of the compound (A) is preferably from 1.01 to 1.50, more preferably from 1.05 to 1.35.

When Mn and Mw/Mn are within such ranges, the viscosity is low, the volatile component is little, and the uniformity is excellent when the compound (A) is dissolved in a liquid medium.

Mn is measured by gel permeation chromatography (hereinafter referred to as GPC). Mw/Mn is determined from Mn and Mw (mass average molecular weight) measured by GPC.

The compound (A) can be produced in such a manner that a compound (F) of which all terminals are the groups (W), or a compound (F) of which some terminals are the groups (Z) and the other terminals are the groups (W), is produced in accordance with the method described in WO 2005/068534, and a part or all of the groups (W) at the terminals of the compound (F) are converted to the groups (X) by a known method.

The compound (F) is specifically produced as follows.

A compound having a FC(O)- group at the terminal thereof is obtained by esterification, liquid phase fluorination or ester decomposition reaction, in accordance with WO 2005/068534. Then, a compound (F) having a HO-CH₂- group at the terminal thereof is obtained by either a method (a) of reacting the compound having a FC(O)- group at the terminal thereof with an alcohol or water to convert the terminal to an ester or a carboxylic acid, followed by reduction, or a method (b) of subjecting a compound having a -C(O)O- group at the terminal obtainable after liquid phase fluorination to an ester-exchange reaction with an alcohol to convert the terminal to an ester, followed by reduction. Further, in the liquid phase fluorination reaction, if its conditions are severe, cleavage of molecular terminals tends to occur. Accordingly, in the liquid phase fluorination reaction, the concentration of fluorine contained in gas blown into the liquid phase is preferably from 5.0 to 50 vol%, more preferably from 10 to 30 vol%.

As the method of converting a part or all of the groups (W) at the terminals of the compound (F) to the groups (X), the following two methods may be mentioned.
(1) A method of introducing a leaving group into the compound (F) to be reacted with a compound containing a hydroxy group.
(2) A method of reacting the compound (F) with a compound containing a hydroxy group.

The alternative choice between the method (1) and the method (2) depends upon the reactivity of the compound containing a hydroxy group. In a case where the compound containing a hydroxy group is e.g. a perfluoro compound having a high reactivity, it is preferred to choose the method (2), and in a case where the compound containing a hydroxy group is e.g. a nonfluorine-containing compound which is less reactive, it is preferred to choose the method (1). Here, as the leaving group in the method (1), -O-SO₂R^{F} (wherein R^{F} is a perfluoroalkyl group) may, for example, be mentioned.

The compound (A-1) may, for example, be produced by the following method.

A method of obtaining the compound (A-1) by reacting a compound (such as R^{F}SO₂F, wherein R^{F} is a perfluoroalkyl group) which can introduce a leaving group, with the compound (F) to introduce the leaving group (such as -O-SO₂R^{F}) into -OH at the terminal of the compound (F) to obtain a compound (G), and reacting phenol with the compound (G).

The compound (A-2) may, for example, be produced by the following method.

A method of obtaining the compound (A-2) by reacting perfluorotoluene with the compound (F).

The compound (A-1) and the compound (A-2) may be purified after the production by the above methods. By purifying the compounds, it is possible to increase the purity. As a means for the purification, it is possible to employ a known means of purifying an organic compound, and column chromatography or supercritical extraction may, for example, be mentioned.

### <Lubricant>

In a case where the ether compound (A) is used as a lubricant, the ether compound (A) may be used as it is, PFPE (hereinafter also referred to as another PFPE) other than the ether compound (A) may be added to the ether compound (A), or the ether compound (A) may be added to such another PFPE.

In order to adequately obtain the properties of the ether compound (A), the proportion of the ether compound (A) in the lubricant is preferably at least 10 mass%, more preferably at least 75 mass%, per 100 mass% of the lubricant (total amount of the ether compound (A) and another PFPE, excluding a liquid medium). The upper limit of the proportion of the ether compound (A) in the lubricant is 100 mass%.

### (Another PFPE)

As such another PFPE, e.g. another PFPE having a hydroxy group at the terminal thereof or another PFPE having an ultraviolet absorbing group at the terminal thereof is preferred.

As such another PFPE having a hydroxy group at the terminal thereof, FOMBLIN Z-DiOL or FOMBLIN Z-TetraOL, manufactured by SOLVAY SOLEXIS, DEMNUM SA, manufactured by DAIKIN INDUSTRIES, LTD, or PFPE described in WO 2005/068534 may, for example, be mentioned.

As such another PFPE having an ultraviolet absorbing group at the terminal thereof, FOMBLIN Z-DIAC, FOMBLIN Z-DEAL, FOMBLIN AM2001 or FOMBLIN Z-DISOC, manufactured by SOLVAY SOLEXIS, DEMNUM SH, manufactured by DAIKIN INDUSTRIES, LTD or Moresco A20H, manufactured by MATSUMURA OIL Co., Ltd. may, for example, be mentioned.

Such another PFPE is preferably one containing no PFPE having a CF₃- group at the terminal thereof.

Further, such another PFPE is preferably one having a number average molecular weight (Mn, a value calculated as a perfluoropolyether) of from 1,000 to 10,000.

In order to adequately obtain the properties of the present invention, the proportion of such another PFPE in a case where such another PFPE is added to the lubricant of the present invention, is preferably at most 10 mass%, more preferably 5 mass%, per 100 mass% of the lubricant (total amount of the ether compound (A) and another PFPE, excluding a liquid medium).

### <Composition for lubricant>

In a case where the ether compound (A) of the present invention is used as a lubricant, it is preferably used as a composition for a lubricant containing the ether compound (A) and a liquid medium. The composition for a lubricant may also contain such another PFPE as the case requires.

The composition for a lubricant may be any one of solution, suspension and emulsion, but a solution is preferred. The composition for a lubricant is applied on the surface of a base material (hereinafter also referred to as a "coating step"), the liquid medium is removed (hereinafter also referred to as a "drying step"), and ultraviolet rays are applied thereon (hereinafter also referred to as an "irradiation step"), whereby a lubricant coating is formed.

The liquid medium is preferably a perfluoroalkylamine (such as perfluorotripropylamine or perfluorotributylamine), a polyfluoroalkane (such as AC-2000, AC-4000 or AC-6000 (manufactured by Asahi Glass Company, Limited), or Vertrel XF (manufactured by DuPont)) or a hydrofluoroether (AE-3000 (manufactured by Asahi Glass Company, Limited), or HFE-7100, 7200 or 7300 (manufactured by 3M)), and a hydrofluoroether is more preferred from the viewpoint of low ozone depletion potential.

The liquid medium is removed by evaporating a part or all of the liquid medium at the same time as coating the surface of the base material, or evaporating a part or all of the liquid medium in the subsequent drying step. Accordingly, the liquid medium is preferably selected from liquid media having a boiling point suitable for the coating step or the drying step. That is, a liquid medium is preferably one having a boiling point of from 20 to 150°C, more preferably from 50 to 100°C.

The concentration of the ether compound (A) in the composition for a lubricant is preferably from 0.0001 to 10 mass%, more preferably from 0.001 to 1 mass%.

The composition for a lubricant may contain a component other than the ether compound (A), another PFPE and the liquid medium (hereinafter referred to as "another component").

Such another component may, for example, be a radical scavenger (such as X-1p (manufactured by Dow Chemicals)).

It is preferred that the composition for a lubricant does not contain metal ions, anions, water, low molecular weight polar components, plasticizers or the like because otherwise, the composition for a lubricant would not show the intended performance.

Ions of metals (such as Na, K, Ca and Al) can form Lewis acid catalysts with anions which catalyze decomposition of PFPEs.

Anions (of F, Cl, NO₂ NO₃, PO₄, SO₄, C₂O₄ and the like) and moisture can corrode the surface of a base material. Therefore, the respective contents are preferably such that Al and Mg are at most 1,000 ppb, Na and K are at most 20,000 ppb, Ca is at most 10,000 ppb, and Fe, Ni, Cu and Zn are at most 100 ppb. It is particularly preferred that F is at most 10,000 ppm, and formic acid, Cl, NO₃, SO₄ and oxalic acid are at most 5,000 ppb. Low molecular weight polar compounds (such as alcohols, plasticizers eluted from resins) can impair the adhesion between a base material and a coating.

The water content of the composition for a lubricant is preferably at most 2,000 ppm.

### <Lubricant coating>

A lubricant coating is formed by coating the surface of a base material with a lubricant, followed by ultraviolet irradiation. Or the lubricant coating is formed by coating the surface of a base material with the composition for a lubricant, drying it, and irradiating the surface with ultraviolet rays as the case requires.

The coating method may be roll coating, casting, dip coating (dipping), spin coating, water casting, die coating, Langmuir-Blodgett film formation or vacuum deposition, and dipping is preferred from the viewpoint of uniformity and productivity of a thin film.

It is preferred that the drying step is carried out under conditions where only the liquid medium evaporates without thermal decomposition of the ether compound (A) and another PFPE. The drying temperature is preferably from 50 to 200°C, and from the viewpoint of protection of a base material, the drying temperature is more preferably from 50 to 150°C. The drying time is preferably from 1 to 60 minutes, and from the viewpoint of workability, the drying time is more preferably from 1 to 5 minutes.

In the irradiation step, ultraviolet rays are applied. The wavelength of the ultraviolet rays are preferably 184 nm, 253 nm or a mixed wavelength thereof.

The irradiation time is preferably from 1 to 120 seconds, more preferably from 3 to 60 seconds, particularly preferably from 5 to 30 seconds.

A base material after irradiation with ultraviolet rays may be washed with a fluorine-containing solvent for the purpose of removing contaminants and an excess of a surface treating agent.

By the ultraviolet irradiation, the group (X) at the terminal of the compound (A) can be aligned on the surface of the base material, whereby adhesion of the compound (A) to the surface of the base material can be increased. Further, the lubricant coating having the group (X) at the terminal of the compound (A) aligned, has a high water repellency and a high oil repellency. Accordingly, it is possible to suppress infiltration of e.g. moisture into the lubricant coating, and it is thereby possible to suppress corrosion or deterioration of a base material. Further, the surface energy of the lubricant coating becomes low, and therefore it is advantageous that the friction coefficient becomes low.

The water contact angle (room temperature) on the surface of the lubricant coating after the ultraviolet irradiation, is preferably at least 70°, more preferably at least 80°, particularly preferably at least 85°.

### <Magnetic recording media>

The ether compound (A) is suitable as a lubricant for magnetic recording media such as hard disks.

A base material for magnetic recording media may be a NiP-plated substrate (such as aluminum or glass) having a primer layer, a recording layer and a carbon protective layer (a DLC film) in this order.

The carbon protective layer preferably has a thickness of at most 5.0 nm, and preferably has an average surface roughness (Ra) of at most 2.0 nm.

A lubricant coating in the magnetic recording media is formed by coating the surface of a base material with a lubricant, followed by ultraviolet irradiation. Or it is formed by coating the surface of a base material with a composition for a lubricant, drying it, followed by ultraviolet irradiation.

Since the surface of the lubricant coating after ultraviolet irradiation has a high water repellency, it is possible to suppress infiltration of moisture into the interior of the magnetic recording media even if the lubricant coating is left to stand at high-temperature under high-humidity environment, and it is thereby possible to maintain high lubricity over a long period of time.

The lubricant coating has a thickness of preferably at most 5.0 nm, more preferably at most 3.0 nm, particularly preferably at most 2.0 nm from the viewpoint of improvement of recording density. It is preferably at least 0.25 nm from the viewpoint of durability.

### <Operation and effect>

A lubricant coating formed by employing the ether compound (A) of the present invention has a high water repellency, and even if the lubricant coating is left to stand at high-temperature under high-humidity environment, it is possible to suppress infiltration of moisture into the interior of the magnetic recording media, and it is possible to maintain high lubricity over a long period of time. The reason for this is not clearly understood but is considered to be attributable to the following mechanism.

That is, the ether compound (A) of the present invention has a group (X0) comprising an aromatic ring structure having conjugated π electrons at the terminal thereof, whereby molecules of the ether compound (A) are strongly bonded to one another or to a base material by π-π stacking, whereby the adhesion increases. Further, the bonding by the π-π stacking is less likely to be replaced by the bonding with water, and therefore it is possible to maintain the adhesion to the base material at a high level even in the presence of water. Further, when the ether compound (A) of the present invention, which is applied on the surface of a base material, is irradiated with ultraviolet lays, a part of the group (X) in the ether compound (A) is cut to generate radicals, whereby such a group (X) is chemically bonded to the surface of the base material, and therefore it is possible to maintain adhesion to the base material at a high level even in the presence of moisture.

### EXAMPLES

Now, the present invention will be described in detail with reference to Examples, but, it should be understood that the present invention is by no means restricted to such specific Examples. In the following:
Tetramethylsilane is abbreviated as TMS,
CCl₂FCClF₂ is abbreviated as R-113,
Dichloropentafluoropropane is abbreviated as R-225,
CClF₂CClFCF₂OCF₂CClF₂ is abbreviated as CFE-419,
Hexafluoroisopropyl alcohol is abbreviated as HFIP, and
Isopropyl alcohol is abbreviated as IPA.

Further, analyses in Examples were respectively carried out at room temperature (25°C).
(NMR analysis)

TMS was used as a standard substance for ¹H-NMR (300.4 MHz, cumulated number; 64).

CFCl₃ was used as a standard substance for ¹⁹F-NMR (282.7 MHz, cumulated number; 128).

A measurement solution was prepared by diluting about 0.3 g of the compound or the composition of the ether compound with a solvent (2.0 g). As the solvent, R-113 (5.0 g) having TMS (0.1 g) or CFCl₃ (0.1 g) added thereto was used. Further, in a case where such a sample was in a state of suspension, a small amount of benzene or perfluorobenzene was gradually added as a dissolution assistant, and measurement was carried out in a state where the measurement solution was completely dissolved.

### (GPC analysis)

Mn and Mw were measured, as a value calculated as a perfluoropolyether, by GPC in accordance with JP-A-2001-208736 under the following conditions, and the molecular weight distribution (Mw/Mn) was determined.

Mobile phase: solvent mixture of R-225 (ASAHIKLIN AK-225SEC Glade 1, manufactured by Asahi Glass Company, Limited) and HFIP (R-225/HFIP = 99/1 in volume ratio)

Analytical column: serially connected two PLgel MIXED-E columns (manufactured by Polymer Laboratories)

Molecular weight standard samples: four perfluoropolyethers having Mw/Mn of less than 1.1 and molecular weights of from 2,000 to 10,000 and one perfluoropolyether having Mw/Mn of at least 1.1 and a molecular weight of 1,300

Mobile phase flow rate: 1.0 mL/min

Column temperature: 37°C

Detector: evaporative light scattering detector

### (Film thickness)

A film thickness (a few nm) of a lubricant coating on a disk was measured by ellipsometry method. This method is a method of examining characteristics of the surface of a material from the polarization properties of oblique-incident reflected light, that is a method of applying light of which polarization state is known, from the light incident side, and calibrating a film thickness from the change in the polarization state, changed by reflection on an object to be measured. As a measurement device, a commercially available scanning ellipsometer (MARY-102, manufactured by Five Lab K.K.) was used, and as a measurement light, an elliptically polarized light (spot size: Φ35 µm × 100 µm) of He-Ne laser was used.

### (Bonding ratio)

The thicknesses of the lubricant coating before and after washing were measured by an ellipsometer. The bonding ratio is a ratio of the thickness of the lubricant coating after washing to the thickness of the lubricant coating before washing, represented by percentage. Further, the washing was carried out by dipping the lubricant coating in a commercially available Vertrel-XF (manufactured by DuPont) at 30°C for 10 minutes.

### (Friction coefficient)

The friction coefficient on the surface of the lubricant coating was measured with a friction meter (Tribogear, manufactured by Heidon) using a SUS ball with a diameter of 10 mm as a contactor under a load of 1 g at 25 rpm.

### (Water contact angle)

Water contact angles on the surface of the lubricant coating were measured with a contact angle meter (CA-X, manufactured by Face). On the surface of the lubricant coating, a water droplet with a volume of about 2 µL was put for measurement. The respective samples were measured five times, and the average was obtained. Further, after preparation of the water droplet, the water contact angles were measured after 5 seconds, 60 seconds and 120 seconds, and the stability of the surface energy was observed.

### [Example 1]

A reaction was carried out in the same manner as in the method disclosed in Example 11 in WO 2005/068534 except that polyoxyethylene glycerol ether (UNIOX G1200, manufactured by NOF CORPORATION) was changed to glycerol-initiated polyoxyethylene ether (SC-E1500, manufactured by SAKAMOTO YAKUHIN KOGYO CO., LTD.). With diglycerol-initiated polyoxyethylene ether, FC(O)CF(CF₃)OCF₂ CF(CF₃)O(CF₂)₃ F was reacted to obtain the following compound (B-1) which was liquid at room temperature. As a result of the NMR analysis, the average value of (b10+b20+b30+b40) in the compound (B-1) was 37.0, R^{f} was -CF(CF₃)OCF₂CF(CF₃)OCF₂CF₂ CF₃, Mn was 2,600, and Mw/Mn was 1.15.

Further, the compound (B-1) had no -OCH₂O- structure or no -OCF₂O- structure in its constitution, and further in the ¹⁹F-NMR (solvent: CDCl₃), no peak of δ was confirmed within a range of from -50.0 to -56.0 ppm. 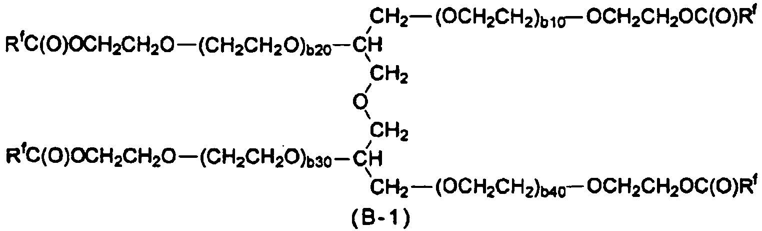 ¹ H-NMR (solvent: CDCl₃) δ (ppm): 3.4 to 3.8, 4.5.
¹⁹ F-NMR (solvent: CDCl₃) δ (ppm): -76.0 to -81.0, -81.0 to -82.0, -82.0 to -82.5, -82.5 to -85.0, -128.0 to -129.2, -131.1, -144.7.

### [Example 2]

A liquid phase fluorination reaction of the compound (B-1) obtained in Example 1 was carried out in the same manner as in the method disclosed in Example 2-1 in WO 2005/068534 except that R-113 was changed to CFE-419, and the concentration of fluorine gas contained in the gas blown to the liquid phase was changed from 20 vol% to 10 vol%. The product was a composition (c-1) containing the following compound (C-1) as the main component and having at least 99.9 mol% of hydrogen atoms in the compound (B-1) substituted by fluorine atoms.

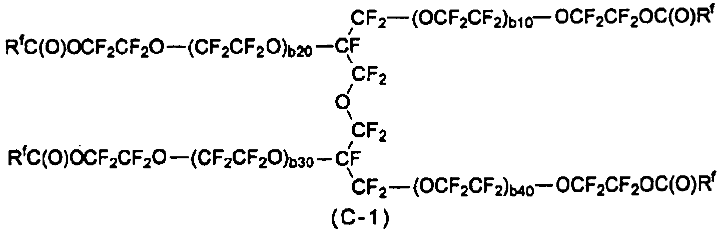

The NMR spectra of the composition (c-1) measured are as follows.
¹ H-NMR δ (ppm): 5.9 to 6.4.
¹⁹ F-NMR δ (ppm): -55.8, -77.5 to -86.0, -88.2 to -92.0, -120.0 to -139.0, -142.0 to -146.0.

### [Example 3]

With respect to the compound (C-1) obtained in Example 2, an ester decomposition reaction was carried out in accordance with the method disclosed in Example 3 in WO 2005/068534 to obtain a composition (d-1) containing the following compound (D-1) as the main component.

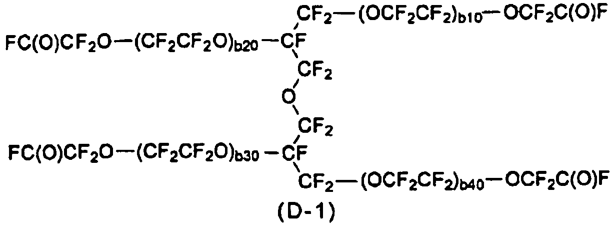

### [Example 4]

### [Example 4-1]

An esterification reaction was carried out by reacting the compound (D-1) obtained in Example 3 with ethanol in accordance with the method disclosed in Example 4-1 in WO 2005/068534. A composition (e-1) containing the following compound (E-1) as the main component was obtained. The composition (e-1) was used for the reaction in Example 5.

### [Example 4-2]

An ester exchange reaction was carried out by reacting the compound (D-1) obtained in Example 3 with ethanol in accordance with the method disclosed in Example 4-2 in WO 2005/068534 to obtain a composition containing the compound (E-1) as the main component.

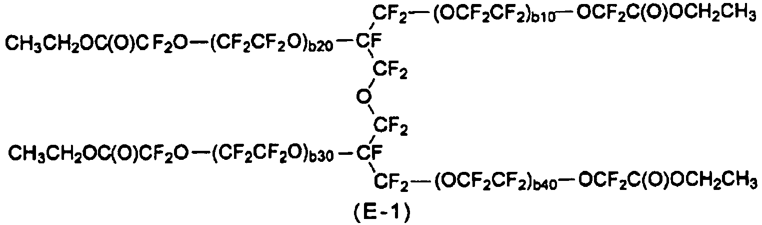

### [Example 5]

A reduction reaction of the compound (E-1) obtained in Example 4-1 was carried out in accordance with the method disclosed in Example 5 in WO 2005/068534 to obtain a composition (f-1) containing the following compound (F-1) as the main component.

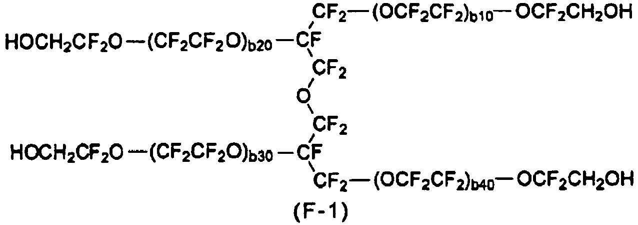

The NMR spectra of the composition (f-1) measured are as follows.
¹ H-NMR δ (ppm): 3.94.
¹⁹ F-NMR δ (ppm): -54.0, -80.1, -88.2 to -90.5, -135.0 to -139.0, -145.5.

### [Example 6]

Examples 1 to 5 were repeated to obtain 150 g of the composition (f-1).

### [Example 6-1]

In 250 mL of a round flask in a nitrogen atmosphere, 100 g of the composition (f-1) obtained in Example 6 and 200 g of R-225 were charged, and stirred to be uniformly mixed. On the round flask, a condenser having an outlet kept at 0°C and further having the interior replaced by nitrogen gas, and a dropping funnel, were provided.

Then, 14 g of triethylamine (manufactured by Kanto Chemical Co., Ltd.) was charged and stirred until the mixture would be uniform, an ice bath was installed for the flask, and while the internal temperature was controlled to be at most 10°C, 33 g of 1,1,2,2,3,3,4,4,4-nonafluoro(n-butane)sulfonyl fluoride was added. Thereafter, the interior of the round flask was gradually returned to room temperature, followed by stirring for 14 hours. An organic layer was washed with a saturated sodium chloride aqueous solution, phase separation into two phases was carried out, and the resulting organic layer was recovered. The organic layer was dried over 2.0 g of magnesium sulfate, and the solvent was distilled off by an evaporator to obtain 41.4 g of a pale yellow composition (g-1) in a form of liquid at 25°C. As a result of the analysis, 90 mol% of the hydroxy group terminals (-CF₂CH₂OH) of the compound (F-1) were substituted with -CF₂CH₂OS(O)₂CF₂CF₂CF₂CF₃, and the product was confirmed to be the composition (g-1) having the compound (G-1) as the main component.

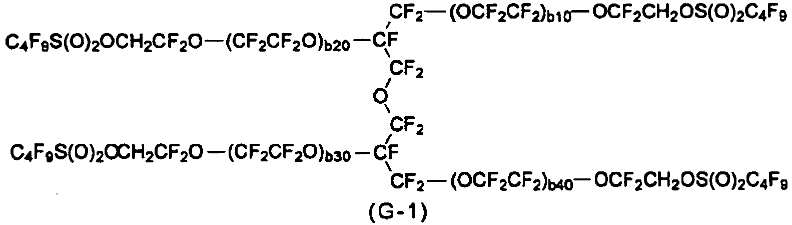

The NMR spectra of the composition (g-1) measured are as follows.
¹ H-NMR δ (ppm): 3.94, 4.60.
¹⁹ F-NMR δ (ppm): -54.0, -77.6, -80.1, -80.9, -88.2 to -90.5, -110.0, -121.0, -126.0, -135.0 to -139.0, -145.5.

### [Example 6-2]

10.92 g of cesium carbonate (manufactured by Kanto Chemical Co., Ltd.), 5.2 g of phenol (manufactured by Kanto Chemical Co., Ltd.) and 200 g of dimethylacrylamide (manufactured by Kanto Chemical Co., Ltd.) were charged in a 500 mL four-necked flask in a nitrogen atmosphere. A stirrer chip was put in the flask, and at the top of the flask, a condenser cooled to 10°C, and a dropping funnel, were equipped. Thereafter, the flask was heated with stirring in an oil bath, and the liquid temperature was kept at 80°C. After the temperature was stabilized, 50 g of the composition (g-1) obtained in Example 6-1 was dropwise added over a period of two hours. After completion of the dropwise addition, the liquid temperature was increased to 100°C, followed by stirring for 3 hours. Then, the temperature was cooled to room temperature, and the reaction fluid was diluted with 250 g of R-225. Washing and liquid separation were carried out twice by 500 mL of a 0.05 N diluted hydrochloric acid solution, and then the resulting organic phase was concentrated by an evaporator to obtain 51.9 g of a pale yellow composition (a-1) in a form of liquid at 25°C. As a result of the analysis, 98 mol% of the terminal groups (-CF₂CH₂OS(O)₂CF₂CF₂CF₂CF₃) of the compound (G-1) were substituted with -CF₂CH₂O-Ph (wherein Ph represents a phenyl group, the same applies hereinafter), and the product was confirmed to be the composition (a-1) having the compound (A-1) as the main component.

Further, since it is confirmed that the compound (B-1) contains no -OCH₂O-structure or no -OCF₂O- structure, it is confirmed that the composition (a-1) also contains no -OCF₂O- structure.

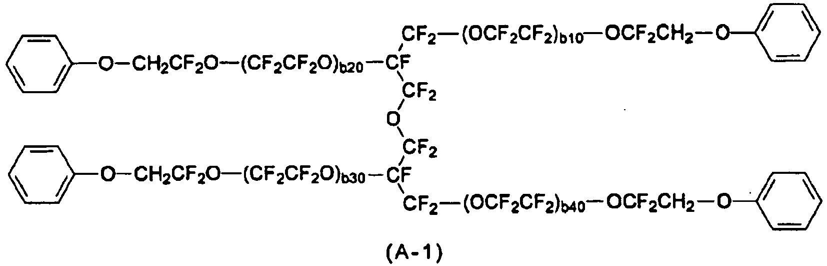

The NMR spectra of the composition (a-1) measured are as follows.
¹ H-NMR δ (ppm): 4.41, 4.75, 7.06, 7.56, 7.68.
¹⁹ F-NMR δ (ppm): -54.0, -77.8, -80.1, -88.2 to -90.5, -135.0 to -139.0, -145.5.

### [Example 6-3]

12.5 g of cesium carbonate (manufactured by Kanto Chemical Co., Ltd.), 50 g of the composition (f-1) and 100 g of dimethylacrylamide (manufactured by Kanto Chemical Co., Ltd.) were charged in a 500 mL four-necked flask in a nitrogen atmosphere. A stirrer chip was put in the flask, and at the top of the flask, a condenser cooled to 10°C, and a dropping funnel were equipped. The flask was heated to 80°C, and then 14.0 g of perfluorotoluene (manufactured by HYDRUS CHEMICAL INC.) was added thereto over a period of one hour from the dropping funnel. After completion of the dropwise addition of perfluorotoluene, the liquid temperature was increased to 100°C and maintained for 3 hours. Then, the temperature was cooled to room temperature, and the reaction fluid was diluted with 250 g of R-225. Washing and liquid separation were carried out twice with 500 mL of a 0.05 N diluted hydrochloric acid aqueous solution, and then the resulting organic phase was concentrated by an evaporator to obtain 51.9 g of a pale yellow composition (a-2) in a form of liquid at 25°C. As a result of the analysis, 92 mol% of terminal groups (-CF₂CH₂OH) of the compound (F-1) were substituted with -CF₂CH₂O-Ph(F)-CF₃ (wherein Ph(F)-CF₃ represents a 2,3,5,6-tetrafluoro-4-trifluoromethyl-phenyl group), and the product was confirmed to be the composition (a-2) having the compound (A-2) as the main component.

Further, since it is confirmed that the compound (B-1) contains no -OCH₂O-structure or no -OCF₂O- structure, it is confirmed that the composition (a-2) also contains no -OCF₂O- structure.

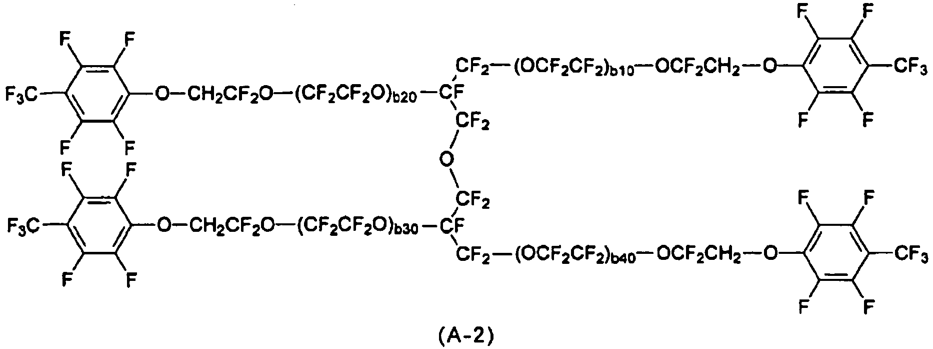

The NMR spectra of the composition (a-2) measured are as follows.
¹ H-NMR δ (ppm): 3.94, 4.48.
¹⁹ F-NMR δ (ppm): -54.0, -55.1, -77.7, -80.1, -88.2 to -90.5, -135.0 to -139.0, -140.0, -145.5, -155.4.

### [Example 7]

Examples 6-1 and 6-2 were repeated to obtain 100 g of the composition (a-1). Further, Example 6-3 was repeated to obtain 100 g of the composition (a-2).

### [Example 7-1]

Each of the composition (a-1) and the composition (a-2) contains a low polar component formed by cleavage of a C-C bond, and a hydroxy group terminal component in which no aromatic ring is introduced. Accordingly, the composition (a-1) and the composition (a-2) were purified by the following column chromatography.

A slurry of a particulate silica gel (MS-Gel D75-120A, manufactured by S.I. Tech Co., Ltd.) in R-225 was packed into a column with a diameter of 150 mm and a length of 500 mm to form a silica gel bed with a height of 100 mm.

The composition was loaded on the silica gel bed, and then a low polar component in the composition was eluted by letting only R-225 as an extraction solvent flow. Then, by using an extraction solvent (solvent mixture of R-225 and HFIP or IPA), the concentration of HFIP or IPA in the extraction solvent was stepwise increased to be 0%, 10% and 50% depending upon the polarity of the terminal group, to elute a desired product, crude products (h1) to (h4) were recovered from the composition (a-1), and crude products (i1) to (i4) were recovered from the composition (a-2). The recovered amount and the proportion ("%" represents "mol%") of the terminal group, of the respective crude products, are shown in Tables 1 and 2.

**TABLE 1**

| | | Extraction solvent (%) | | Mass (g) | Terminal group (% NMR) | | |
|---|---|---|---|---|---|---|---|
| | | R225 | IPA | | Group (X) | Group (Z) | Group (W) |
| Charge | a-1 | - | - | 100 | 89 | 2 | 9 |
| Recovery | h1 | 100 | 0 | 2 | 50 | 50 | 0 |
| | h2 | 90 | 10 | 7 | 65 | 33 | 2 |
| | h3 | 50 | 50 | 74 | 95 | 1 | 4 |
| | h4 | 50 | 50 | 16 | 75 | 0 | 25 |

**TABLE 2**

| | | Extraction solvent (%) | | Mass (g) | Terminal group (% NMR) | | |
|---|---|---|---|---|---|---|---|
| | | R225 | P HFIP | | Group (X) | Group (Z) | Group (W) |
| Charge | a-2 | - | - | 100 | 92 | 2 | 6 |
| Recovery | i1 | 100 | 0 | 10 | 55 | 45 | 0 |
| | i2 | 90 | 10 | 72 | 94 | 1 | 5 |
| | i3 | 90 | 10 | 12 | 75 | 1 | 25 |
| | i4 | 50 | 50 | 3 | 50 | 0 | 50 |

### [Example 7-2]

Each of the crude products obtained in Example 7-1 has a molecular weight distribution. Accordingly, the crude products (h3) and (i2) were purified by the following supercritical extraction method.

Each crude product was packed in a pressure-resistant extraction bath having an internal capacity of 200 mL and heated to 60°C, and carbon dioxide in a supercritical state was flown at a flow rate of 10 cc/min to carry out extraction purification. While the pressure of carbon dioxide was stepwise increased from 10 to 25 MPa depending upon the molecular weight, a desired product was eluted, and purified products (h3-1) to (h3-4) were recovered from the crude product (h3) and purified products (i2-1) to (i2-4) were recovered from the crude product (i2). The recovered amount and the proportion of the terminal group, of the respective purified products are shown in Tables 3 and 4.

**TABLE 3**

| | | Extraction condition (MPa) | Mass (g) | Terminal group (% NMR) | | | GPC analysis value | |
|---|---|---|---|---|---|---|---|---|
| | | | | Group (X) | Group (Z) | Group (W) | Mn | Mw/Mn |
| Charge | h3 | - | 70 | 95 | 1 | 4 | 2,450 | 1.28 |
| Recovery | h3-1 | 10 | 10 | 87 | 3 | 10 | 1,940 | 1.10 |
| | h3-2 | 20 | 21 | 98 | 0 | 2 | 2,340 | 1.13 |
| | h3-3 | 22 | 25 | 99 | 0 | 1 | 2,660 | 1.15 |
| | h3-4 | 25 | 13 | 100 | 0 | 0 | 3,100 | 1.20 |

**TABLE 4**

| | | Extraction condition (MPa) | Mass (g) | Terminal group (% NMR) | | | GPC analysis value | |
|---|---|---|---|---|---|---|---|---|
| | | | | Group (X) | Group (Z) | Group (W) | Mn | Mw/Mn |
| Charge | i2 | - | 70 | 94 | 1 | 5 | 2,150 | 1.25 |
| Recovery | i2-1 | 10 | 5 | 97 | 3 | 0 | 1,620 | 1.08 |
| | i2-2 | 12 | 25 | 100 | 0 | 0 | 2,070 | 1.12 |
| | i2-3 | 15 | 26 | 95 | 0 | 5 | 2;330 | 1.15 |
| | i2-4 | 20 | 12 | 85 | 0 | 15 | 2,870 | 1.19 |

### [Example 8] (Comparative Example)

In accordance with the method disclosed in Examples 1 to 7 of JP-A-2009-149835, a composition (a4-1) containing, as the main component, a compound (A4-1) having three vinyl groups at the terminals thereof, was obtained. This composition was purified by column chromatography to obtain a purified product (j-1).

### [Example 9]

### [Example 9-1]

The purified products (h3-2), (h3-3), (i2-2) and (i2-3), and the composition (f-1) and the purified product (j-1) as comparison, were respectively diluted with a liquid medium (Vertrel-XF, manufactured by DuPont) to prepare 6 types of compositions for a lubricant having a concentrations of 0.01 mass%.

### [Example 9-2]

Using carbon as a target, DLC was vapor-deposited on glass blanks (2.5" blanks, manufactured by Asahi Glass Company, Limited) for magnetic disks by radiofrequency magnetron sputtering in an argon atmosphere to form a DLC film thereby to prepare a simulated disk. The gas pressure of argon was 0.003 Torr, and the electric power density during the sputtering was 3 W/cm² per target area. The thickness of the DLC film was 30 nm. The water contact angle of the surface of the DLC film was 40°.

### [Example 9-3]

In the composition for a lubricant, obtained in Example 9-1, the simulated disk obtained in Example 9-2 was immersed for 30 seconds, and lifted up at a constant rate of 6 mm/sec. Thereafter, the simulated disk was put on a hot plate at 100°C for 60 minutes to remove the liquid medium. Using an ultraviolet irradiating apparatus (UV CrossLinker CX-2000, manufactured by UVP), the simulated disk coated with the composition for a lubricant in a state where the liquid medium was removed, was irradiated with ultraviolet rays to form a lubricant coating, The wavelength of the ultraviolet rays was a mixed wavelength of 184 nm and 253 nm, and the irradiation time was 15 seconds.

The disk obtained was immersed in a solvent (Vertrel-XF) and washed therewith for 30 seconds to produce a simulated hard disk. The bonding property, the friction coefficient and the water contact angle of the lubricant coating on the surface of the simulated hard disk obtained are shown in Tables 5 and 6.

Further, a lubricant coating was formed in the same manner except that no ultraviolet irradiation was carried out. The bonding property, the friction coefficient and the water contact angle of the lubricant coating on the surface of the disk are shown in Tables 5 and 6.

**TABLE 5**

| Lubricant | Film thickness (Å) | Bonding ratio (%) | | Friction coefficient (-) | |
|---|---|---|---|---|---|
| | | Without UV | With UV | Without UV | With UV |
| h3-2 | 10 | 52 | 78 | 3.24 | 2.17 |
| h3-3 | 11 | 60 | 80 | 3.56 | 2.01 |
| i2-2 | 8 | 48 | 65 | 2.78 | 1.98 |
| i2-3 | 9 | 50 | 67 | 2.88 | 2.05 |
| f-1 | 15 | 75 | 78 | 2.33 | 2.13 |
| j-1 | 14 | 62 | 80 | 4.00 | 3.59 |

**TABLE 6**

| Lubricant | Water contact angle (°) | | | | | |
|---|---|---|---|---|---|---|
| | Without UV | | | With UV | | |
| | After 5 seconds | After 60 seconds | After 120 seconds | After 5 seconds | After 60 seconds | After 120 seconds |
| h3-2 | 62 | 59 | 56 | 85 | 83 | 81 |
| h3-3 | 64 | 61 | 57 | 83 | 82 | 82 |
| i2-2 | 75 | 65 | 60 | 90 | 89 | 87 |
| i2-3 | 76 | 67 | 60 | 92 | 90 | 89 |
| f-1 | 72 | 62 | 53 | 75 | 65 | 55 |
| j-1 | 61 | 53 | 48 | 70 | 55 | 49 |

With respect to the simulated hard disks produced by using the lubricants (h3-2), (h3-3), (i2-2) and (i2-3) as Examples, it was confirmed that the friction coefficient was low, and change with time of water contact angle was small even if water was infiltrated therein, that is, it is possible to maintain the adhesion to a base material at a high level even in the presence of moisture. Further, from the measurement result of the water contact angle, it is found that the water contact angle of a coating film comprising a lubricant having an aromatic ring at the terminal thereof becomes large by the ultraviolet irradiation treatment, that is, the surface energy readily lowers, as compared with a coating film comprising a lubricant having only a hydroxy group at the terminal thereof.

Further, it is found that a strong bond which is not replaced by a polar compound is formed between the lubricant having an aromatic ring at the terminal thereof and the disk. Especially, of the coating film comprising a lubricant having a fluorinated aromatic ring at the terminal thereof, the surface energy is sufficiently low and the friction coefficient is sufficiently small.

On the other hand, in the case of the simulated hard disks produced by using the lubricants (f-1) and (j-1) as Comparative Examples, even though the bonding ratio was good, from the measurement result of the water contact angle, it is found that the change with time of the water contact angle became large when water was infiltrated therein.

### INDUSTRIAL APPLICABILITY

The ether compound of the present invention is useful as a lubricant for magnetic recording media such as hard disks.

The entire disclosure of Japanese Patent Application No. 2009-268683 filed on November 26, 2009 including specification, claims and summary is incorporated herein by reference in its entirety.

## Claims

1. An ether compound represented by the following formula (A):
(X-)ₛ (W-)ₜ (Z-)ᵤ Y (A)
wherein X is a group (X) having a group represented by the following formula (X0) at the terminal thereof,
W is a group (W) having a group represented by the following formula (W0) at the terminal thereof,
Z is a group (Z) having a group represented by the following formula (Z0) at the terminal thereof,
Y is a group (Y) which is a (s+t+u) valent perfluorinated hydrocarbon group or a group having an etheric oxygen atom inserted between carbon-carbon atoms of the hydrocarbon group,
s is an integer of from 1 to 7,
t is an integer of from 0 to 4,
u is an integer of from 0 to 2, and
(s+t+u) is an integer of from 2 to 7: 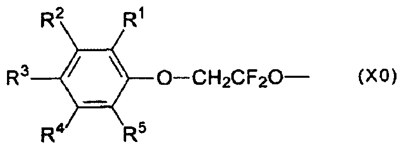
HO-CH₂CF₂O- (W0)
CF₃(CF₂)ₐ₃O- (Z0)
wherein among R¹ to R⁵, two of them are each independently an atom or group selected from a hydrogen atom, a fluorine atom, a methyl group, a trifluoromethyl group, a phenyl group, a perfluorophenyl group and a hydroxy group, and other three are each independently a hydrogen atom or a fluorine atom, and
a3 is an integer of from 0 to 19.

2. The ether compound according to Claim 1, wherein the group (X) is a group represented by the following formula (X1), the group (W) is a group represented by the following formula (W1), and the group (Z) is a group represented by the following formula (Z1): 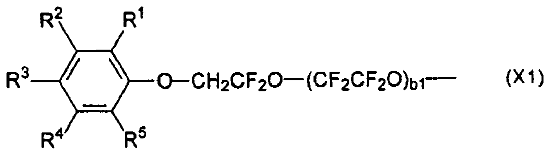
HO-CH₂CF₂O-(CF₂CF₂O)_{b2}- (W1)
CF₃(CF₂)ₐ₃O-(CF₂CF₂O)_{b3}- (Z1)
wherein b1 to b3 are each independently an integer of from 3 to 200.

3. The ether compound according to Claim 2, wherein the group (X1) is a group selected from the group consisting of groups represented by the following formulae (X1-1) to (X1-5):

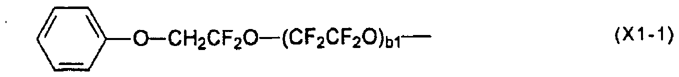

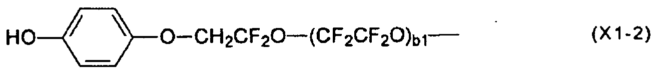

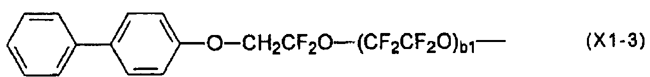

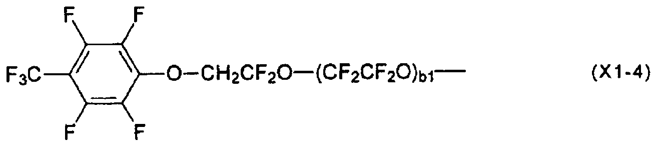

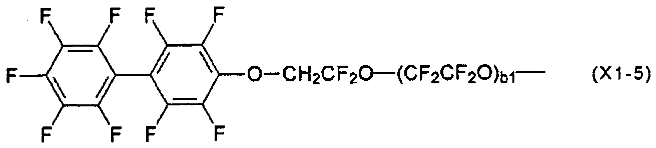

4. The ether compound according to any one of Claims 1 to 3, wherein the group (Y) is a group selected from the group consisting of groups represented by the following formulae (Y-1) to (Y-8):

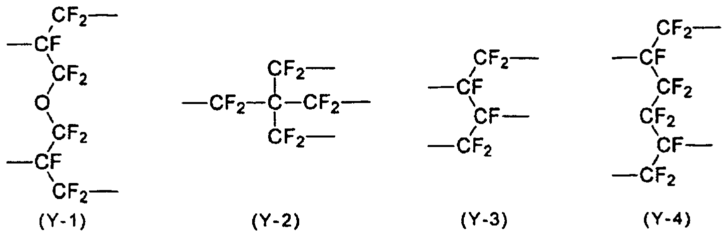

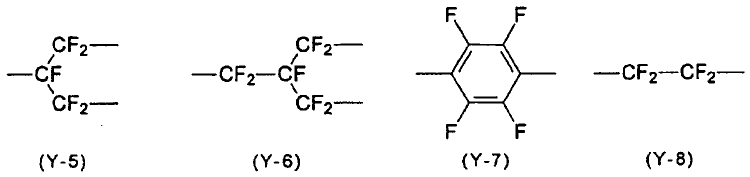

5. The ether compound according to any one of Claims 1 to 4, which has no -OCF₂O- structure.

6. The ether compound according to any one of Claims 1 to 5, wherein the average value of s/(s+t+u) is from 0.25 to 1.0.

7. The ether compound according to any one of Claims 1 to 6, wherein the number average molecular weight (a value calculated as a perfluoropolyether) is from 500 to 1,000,000, and further the molecular weight distribution (a value calculated as a perfluoropolyether) is from 1.01 to 1.50.

8. A lubricant containing the ether compound as defined in any one of Claims 1 to 7, wherein the proportion of the ether compound is at least 10 mass%, per 100 mass% of the lubricant (excluding a liquid medium).

9. The lubricant according to Claim 8, which is used for magnetic recording media.

10. A composition for a lubricant, comprising the ether compound as defined in any one of Claims 1 to 7 and a liquid medium.

11. The composition for a lubricant according to Claim 10, wherein the proportion of the ether compound is from 0.0001 to 10 mass%, per 100 mass% of the composition for a lubricant.
